(19)

**Europäisches Patentamt**

**European Patent Office**

**Office européen des brevets**

(11) **EP 4 160 199 B1**

(12) **EUROPEAN PATENT SPECIFICATION**

(45) Date of publication and mention
of the grant of the patent:
**12.02.2025 Bulletin 2025/07**

(21) Application number: **21306384.5**

(22) Date of filing: **04.10.2021**

(51) International Patent Classification (IPC):
**G01N 27/327** $^{(2006.01)}$     **B01L 3/00** $^{(2006.01)}$
**C12Q 1/6825** $^{(2018.01)}$

(52) Cooperative Patent Classification (CPC):
**G01N 27/3276; B01L 3/50853; G01N 27/3278;**
B01L 2200/0663; B01L 2200/0668; B01L 2200/12;
B01L 2300/0645; B01L 2300/0893;
B01L 2400/0415; C12Q 1/6825

(54) **APPARATUS FOR BIOMOLECULE ANALYSIS WITH A WELL AND A CAVITY BELOW THE WELL**

VORRICHTUNG ZUR BIOMOLEKÜLANALYSE MIT EINER VERTIEFUNG UND EINEM DARUNTER
BEFINDLICHEN HOHLRAUM

APPAREIL D'ANALYSE DE BIOMOLÉCULES À L'AIDE D'UN PUITS ET D'UNE CAVITÉ
AU-DESSOUS DU PUITS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.04.2023 Bulletin 2023/14**

(73) Proprietor: **Depixus**
**75014 Paris (FR)**

(72) Inventor: **BAI, Hua**
**HARSTON, CB22 7QT (GB)**

(74) Representative: **Regimbeau**
**20, rue de Chazelles**
**75847 Paris Cedex 17 (FR)**

(56) References cited:
**EP-A1- 3 090 803      US-A1- 2003 003 609**
**US-A1- 2009 221 431      US-A1- 2011 155 586**
**US-A1- 2013 281 325      US-A1- 2019 178 840**

## Description

TECHNICAL FIELD

The invention relates to an apparatus and a process for analyzing biomolecules through force spectroscopy. The invention applies in particular to the analysis of nucleic acid molecules such as DNA or RNA or proteins.

BACKGROUND

If biomolecules are attached to micro-scale beads, information about the structure of the biomolecules can be inferred by manipulating the beads and tracking their position with high resolution. For example, a nucleic acid molecule attached to a micro-scale bead can inform on the base sequence, the presence of biochemical modifications to the nucleic acid bases, and the interactions of the nucleic acid molecule with proteins such as polymerases, helicases, topoisomerase, etc.

Under particular experimental conditions, tracking the location of the bead in real time can be used to generate useful information about the structure of the DNA or RNA molecule to which it is attached. This can, in turn, be used to determine the molecule's gross organization, base sequence, the presence of biochemical modifications to the nucleic acid bases, and the interactions of the molecule with proteins such as polymerases, helicases, topoisomerases, etc.

For example, European patent EP3090803 discloses an apparatus for analyzing nucleic acid molecules, comprising: a bead on which one molecule is anchored at one end, a surface on which the molecule is anchored at the other end, an actuator adapted to cause the bead to move relative to said surface in one direction of motion, and a sensor adapted to measure a distance between the bead and the surface. The apparatus also comprises a well having an axis extending along the direction of motion of the bead and a bottom which is formed by said surface. The well is filled with an electrically conductive solution, and the bead being received in the well. The sensor is adapted to measure an impedance of the well, said impedance depending on a distance between the bead and the surface, in view of determining, from the measured impedance, the distance between the bead and the surface, and hence changes in the extension of the molecule with an accuracy up to the nanometer. From this information, the behavior of the molecule under various conditions can be inferred.

Typically, the control of the motion of the bead may rely on a magnetic force applied by the actuator on the bead. For example, the actuator comprises at least one magnet mounted to be displaceable along the direction of the axis of the well. The bead is made in a paramagnetic material, and is interposed between the bottom of the well and the magnet. The magnet allows a magnetic force to apply on the bead and consequently on the molecule to which it is anchored.

The sensor comprises a top electrode, a well and a bottom electrode. The top electrode is positioned over the top surface of the well, in contact with the electrically conductive solution. The top electrode is submitted to a known potential. The bottom electrode is positioned at the bottom surface of the well. An electronic circuit is provided to measure a current flowing between the electrodes. The current circulating between the electrodes is the measured signal.

The signal's strength depends on a ratio between the bead diameter and the microwell diameter at the bead's level. The cross-sectional area of the well perpendicular to the axis increases from the bottom surface to the top surface of the well. Thus, the strength of the signal indicates the depth of the bead within the well, which in turn indicates the molecule's extension.

However, the measured signal is weak, especially where the measurement precision is fundamentally limited by kT/C noise of the double layer capacitance on the bottom electrode.

Accordingly, there is a need for an apparatus for analyzing biomolecules using a biomolecule attached to a bead in a well, which allows a reduction of noise such as kT/C noise of the double layer capacitance and thermal noise of the well and thereby allows an increase in the measurement precision. Increasing precision reduces the number of measurement cycles required for statistical averaging, thereby allowing a reduced total measurement time, which helps achieve a cost-effective system with increased throughput.

SUMMARY OF THE INVENTION

The invention relates to an apparatus for biomolecule analysis comprising a plurality of cells, each cell comprising:

- a bottom surface and a top surface opposite the bottom surface, an axis extending between the bottom surface and the top surface;
- a well extending along the axis from the top surface towards the bottom surface, wherein the well has a cross-sectional area perpendicular to the axis, which monotonically varies along the axis of the well between a minimum and a maximum;
- a molecule anchored at a first end to the bottom surface;
- a bead to which a second end of the biomolecule is anchored, the bead disposed within the well at a position;
- a electrically conductive solution filling the well;
- a top electrode above the well, and a bottom electrode at the bottom surface, both the top electrode and the bottom electrode in contact with the electrically conductive solution,

wherein the apparatus comprises a sensor configured to measure an impedance between the top electrode and the bottom electrode, the impedance depending on the depth of the bead within the well, wherein the cell further comprises a cavity wider than the well is arranged between the bottom electrode and the well, said cavity in fluid communication with the well and exposing the bottom electrode at the bottom surface to the electrically conductive solution filling the cavity.

Other preferred, although non-limitative, aspects of the apparatus are as follows, isolated or in a technically feasible combination:

- the well is at least twice as deep as the cavity along the axis;
- the cross-sectional area of the well decreases in a direction from the top surface to the bottom surface until a minimum is reached at a transition between the well and the cavity;
- a cross-sectional area of the cavity is at least four times a maximum cross-sectional area of the well;
- the well is rotationally symmetrical about the axis and the cavity has a cylindrical shape;
- the cavity has a height along the axis comprised between 0.05 $\mu$m and 1 $\mu$m;
- the apparatus comprises an actuator configured to cause the bead to move relative to the bottom surface along the axis of the well.

In a second aspect, the invention also relates to a process for manufacturing an apparatus as disclosed in the first aspect, said process comprising:

a) forming a bottom electrode on a dielectric substrate to form the bottom surface,
b) depositing a first layer of a first material onto the bottom electrode,
c) depositing a second layer of a second material covering the first material of the first layer, with the second material being different from the first material,
d) forming the well in the second layer of the second material, thereby exposing an area of first material,
e) forming the cavity by etching first material through the well,
f) forming the top electrode,
g) filling the well and cavity with the electrically conductive solution, anchoring the biomolecule at a first end to the bottom surface and at a second end to the bead.

Step b) of depositing a first layer of a first material on the bottom electrode may be followed by a step b1) in which the first material of the first layer is removed except for a volume of first material above the bottom electrode, and wherein at step e) said volume of first material is etched to form the cavity. Dimensions of the cavity may be defined by the volume of first material left by the removal of the first material of the first layer in step b1). The cavity may be defined in the second layer of second material.

BRIEF DESCRIPTION OF THE DRAWINGS

Other aspects, objects and advantages of the present invention will become better apparent upon reading the following detailed description of preferred embodiments thereof, given as non-limiting examples, and made with reference to the appended drawings wherein:

- Figure 1 shows a cross-sectional view of a cell of the apparatus, in accordance of a possible embodiment of the invention,
- Figures 2a to 2f show steps of an example of manufacturing process to obtain an apparatus with a cell as illustrated in Figure 1, in accordance of a possible embodiment of the invention,
- Figures 3a to 3g show steps of an example of manufacturing process to obtain an apparatus with a cell as illustrated in Figure 1, in accordance of a possible embodiment of the invention.

DETAILED DESCRIPTION OF THE INVENTION

The apparatus includes a plurality of cells, each cell configured to carry out an analysis of a biomolecule. A biomolecule or biological molecule, is a molecule present in organisms that are essential to one or more typically biological processes. Typically, a biomolecule is a nucleic acid such as DNA or RNA, or a protein or complex of protein. Preferably, the biomolecule may be a double-stranded molecule of the hairpin type. Hairpin means a double helix wherein the 5' end of one strand is physically linked to the 3' end of the other strand through an unpaired loop. This physical link can be either covalent or non-covalent, but is preferably a covalent bond. A hairpin thus consists of a double-stranded stem and an unpaired single-stranded loop. The biomolecule can also have other shapes, and especially may have some other reversible conformational change. For example, in the case of a protein, the biomolecule can be folded or unfolded.

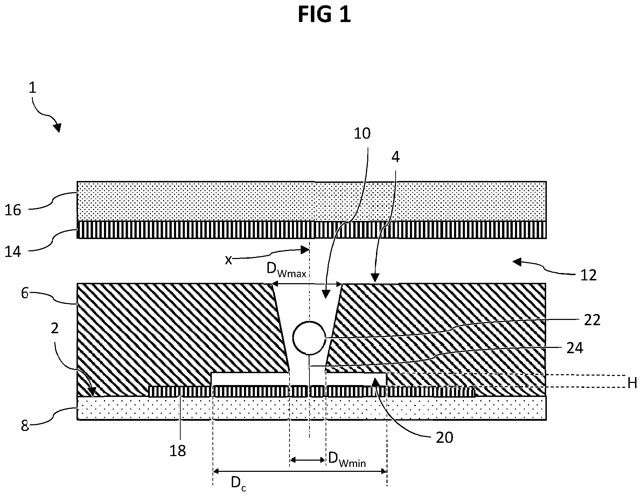

The apparatus may contain several hundreds or thousands of wells, up to several tens of millions or hundreds of millions, arranged in a planar pattern. The number of cells may preferably be greater than several thousands or millions, preferably greater than ten million (10,000,000), for instance greater than one hundred million (100,000,000). Since the cells share the same structure, only one cell will be described. With reference to **Figure 1**, each cell 1 includes a bottom surface 2 and a top surface 4 opposite the bottom surface 2. An axis X extends between the bottom surface 2 and the top surface 4, and is perpendicular to the bottom surface 2 and the top surface 4.

The top surface 4 is the surface of a main layer 6 of electrically insulating material, for example a semiconductor such as silicon, glass, a non-conducting polymer such as polyimide, an organic dielectric, or a resin. The layer 6 is formed on a substrate 8 also made of electrically insulating material. In this example, the interface between the layer 6 and the substrate 8 forms the bottom surface 2.

A well 10 extends along the axis X from the top surface 4 towards the bottom surface 2. The well 10 opens at the top surface 4 on a channel 12. The well 10 forms a void in the main layer 6. The well 10 is a so-called microwell, because the order of magnitude of their dimensions (depth, largest length of a cross-section) is about 1 μm or about 0.1 μm. For instance, a well 10 can have a depth along the axis X of a few micrometers, for instance comprised between 1 and 10 micrometers, for instance equal to 8 μm.

The well 10 has a cross-sectional area perpendicular to the axis X, which monotonically varies along the axis X between a minimum and a maximum. Typically, the cross-sectional area of the well 10 decreases in a direction from the top surface 4 to the bottom surface 2. The cross-sectional area of the well 10 may otherwise increase in a direction from the top surface 4 to the bottom surface 2.

Preferably, the well 10 is rotationally symmetrical about the axis X. Viewed in a plane containing the axis X, the sidewall of the well 10 is not parallel with the axis X. The sidewall angle of the wall 10 is preferably comprised within 70°-89° with respect to a plane perpendicular to the axis X. A diameter of a cross-section of the well 10 in a plane orthogonal to the axis X varies between a minimum diameter $D_{Wmin}$ and a maximum diameter $D_{Wmax}$. A largest length or diameter of a cross-section of the well 10 in a plane orthogonal to the axis X can range from a few hundred nanometers to a few micrometers, for instance about 4 or 5 μm. As in Figure 1, the well 10 may have a frustoconical shape (a truncated cone). Alternatively, the radius of the wall of the well 10 may be defined according to the distance z from the bottom surface 2 by an equation such as:

$$z = l_0 tanh2(r - r_0)$$

where $l_0$ is the height of the well 10 and $r_0$ is the bottom radius of the well 10.

The well 10 and channel 12 are filled with an electrically conductive solution. The electrically conductive solution preferably has a conductivity of between $10^{-7}$ S/cm and 10 S/cm, preferably between $10^{-4}$ S/cm and $10^{-1}$ S/cm, and more preferably between 1 to 20 mS/cm. For instance, the solution may be an aqueous solution of sodium chloride at a concentration of 100 mmol/m³ (100 mM). The solution may alternatively comprise a buffer compatible with the preservation of DNA molecules, such a buffered aqueous solution containing 10mM Tris HCl and 0.1 mM EDTA and sodium Chloride at 100mM. The buffer may also contain divalent cations compatible with enzymatic activities, such a 10 mM MgCl2. In some embodiments, the buffer should support electrophoresis (e.g. Tris Borate EDTA buffer).

A top electrode 14 is above the well 10. In this example the top electrode 14 is separated from the top surface 4 by the channel 12, but the top electrode 14 may also be arranged at the top surface 4. The top electrode 14 is for example supported above the well 10 by a support plate 16. The top electrode 14 is in contact with the electrically conductive solution. A bottom electrode 18 is arranged at the bottom surface 2, typically on the surface of the substrate 8. The bottom surface 2 is therefore formed by the bottom electrode 18 and by the surface of the substrate 8 where the bottom electrode 18 is absent. The top electrode 14 and the bottom electrode 18 are for example gold or platinum electrodes, but may be of any other suitable material.

A cavity 20 wider than the well 10 is arranged between the bottom electrode 18 and the well 10. The cavity 20 is in fluid communication with the well 10 and therefore continues the void of the well 10 in the main layer 6 toward the bottom surface 2, and more specifically to the bottom electrode 18 arranged at the bottom surface 2. The cavity 20 is also filled with the electrically conductive solution. The bottom electrode 18 is larger than the cavity 20, so that the bottom electrode 18 forms a bottom for the cavity 20. The cavity 20 exposes the bottom electrode 18 at the bottom surface 2 to the electrically conductive solution filling the cavity 20. A large area of the bottom electrode 18 is thus in contact with the electrically conductive solution.

The depth or height H of the cavity 20 along the axis X is much smaller than the depth of the well 10 along the axis X. Preferably, the cavity 20 has a height H along the axis X comprised between 0.05 μm and 1 μm, preferably between 0.1 μm and 0.8 μm and more preferably comprised between 0.2 μm and 0.5 μm. The well 10 is at least twice as deep as the cavity 20 along the axis X, and preferably the well 10 is at least four to twenty times deeper than the cavity 20. For example, the well has a height along the axis X greater than 1 μm, preferably greater than 2 μm, and more preferably greater than 5 μm.

The cavity 20 is however much wider than the well 10. The cross-sectional area of the cavity 20, perpendicular to the axis X, is at least twice a maximum cross-sectional area of the well 10, and preferably at least four times a maximum cross-sectional area of the well 10. For example, the cavity 20 has a largest cross-section perpendicularly to the axis X comprised between 2 μm and 7 μm. If the cross sections of the well 10 and the cavity 20 are circular, the diameter $D_c$ of the cavity 20 is at least twice the maximal diameter $D_{Wmax}$ of the well 10. This is the case in the illustrated example of **Figure 1,** wherein the well 10 has a frustoconical shape and the cavity 20 has a cylindrical shape. As in this example, the cross-sectional area of the well 10 may decrease in a direction from the top surface 4 to the bottom surface 2 until a minimum is reached at a transition between the well 10 and the cavity 20.

The cell 1 includes a bead 22 disposed within the well 10 at a position along the axis X. The bead 22 is of a spheroid shape, and is preferably spherical, even though it can be oval. The bead 22 has a diameter smaller than the diameter of the cross-section of the well 10, but preferably close to the smaller diameter $D_{Wmin}$ of the cross-section of the well 10. Typically, the bead 22 has a diameter not greater than 5 $\mu$m. For instance, the bead 22 may have a diameter of about 1.5 $\mu$m or 1 $\mu$m. Preferably, the bead may be even smaller and have a diameter of less than 1 um, for instance of 0.3 $\mu$m. The bead 20 must have a different conductivity than the conductivity of the solution. The bead is preferably electrically insulating.

To perform an analysis of a biomolecule, a biomolecule 24 is anchored to the bead 22 at a first end thereof, and to the bottom surface 2 on a second end thereof. Preferably, the second end of the biomolecule 24 is opposite the first end of the biomolecule 24, i.e. the second end on the other side of the molecule with respect to the first end. Since the bottom surface 2 below the well 10 and cavity 20 is formed by the bottom electrode 18, the second end of the biomolecule 24 is anchored to the bottom electrode 18.

To achieve the anchoring of the biomolecule 24 on the bead 22 and on the bottom surface 2, the bead 22 and the bottom surface 2 may be coated with a specific material adapted to bind with an end of the biomolecule 24. For instance, the DNA or RNA molecules may be labelled with biotin at one end, digoxigenin (Dig) at another end, and the beads may be coated with streptavidin to bind with a labelled (for example biotin) end of a DNA / RNA hairpin molecule, and the bottom surface 2, for example the exposed bottom electrode 18, may be further coated with anti-Dig antibodies to bind a Dig-labelled end of the DNA / RNA molecule, see for instance Hunter MM, Margolies MN, Ju A, Haber E, "High-affinity monoclonal antibodies to the cardiac glycoside, digoxin", Journal of Immunology, 1982 Sep; 129(3): 1165-1172.

The bead 22 is attached to the bottom surface 2 via the biomolecule 24, but the bead 22 is moveable relative to the bottom surface 2. In particular, the bead 22 can move in the well 10 along the direction of the axis X. The depth of the position of the bead 22 within the well 10 can thus change, depending on the forces applying on the bead 22.

In order to apply a force on the bead 22 along this axis X, the cell 1 may further include an actuator adapted to cause the bead 22 to move in translation along said axis. The control of the motion of the bead 22 may rely on a magnetic force applied by the actuator on the bead 22. In that case, the bead 22 is made of a paramagnetic material, such as a superparamagnetic material. For instance, the bead 22 may be made in latex with incorporated ferrites, and coated with streptavidin for anchoring the biomolecule 24. The actuator may include at least one permanent magnet, which can be controlled to move in translation along the axis X. The actuator may include two permanent magnets, positioned at equal distance of the axis X and having their magnetic poles aligned perpendicular to the axis X, the North pole of a magnet facing the South pole of the other. The bead 22 is then positioned between the bottom surface 2 and the magnets. These magnets allow one to apply a force on the bead 22 and consequently on the biomolecule 24 to which it is anchored. By moving the magnets closer to or further from the bead 22 along the axis X, one changes the magnetic field and thus controls the magnitude of the force applied to the bead 22, thus controlling the stretching of the biomolecule 24 in the direction of the axis X.

The force applied to the bead 22 can be created by other configurations, in alternative or complement. The actuator may include a permanent magnet and a strip covered with a magnetizable material positioned at a fixed distance relative to the well 10, of about a few micrometers. By bringing the permanent magnet closer or further from the strip covered with magnetizable material the field applied by said strip on the bead 22 can be varied. The actuator may include the top electrode 14 and bottom electrode 18. Other ways of controlling the force applied to the bead 22 can be used, such as optical or acoustic tweezers, the latter implying application of acoustic waves on the bead, see for instance G. Sitters, D. Kamsma, G. Thalhammer, M. Ritsch-Marte, E. J. G. Peterman and G. L. J. Wuite, "Acoustic Force spectroscopy", in Nature Methods, Vol. 12, N°1, Jan. 2015 , or X. Ding, Z. S. Lin, B. Kiraly, H. Yue, S. Li, I. Chiang, J. Shi, S. J. Benkovic and T. J. Huang, "On-Chip Manipulation of single microparticles, cells, and organisms using surface acoustic waves", PNAS, July 10, 2012, vol. 109, n°. 28, 11105-11109.

The apparatus allows determining the position of the bead 22 within the well 10, for example the depth of the bead 22, or the distance between the bead 22 and the bottom surface 2 corresponding to the length of the biomolecule 24 anchored to the bead 22 and the bottom surface 2, by monitoring the impedance, in particular the resistance (or conductance) between the bottom electrode 18 at the bottom surface 2 and the top electrode 14.

Since the well 10 has a cross-section that varies with the depth or the distance along the axis X from the bottom surface 2, and as the bead 22 is of constant size, the bead 22 occupies a varying proportion of the internal volume of the well 10. As an example, in reference to **Figure 1** in which the area of a cross-section of the well 10 perpendicular to the axis X strictly increases with the distance from the bottom surface 2, if the bead 22 is very close to the bottom of the well 10, it occupies a major proportion of the portion of the well 10 extending around the bead 22. The resistance of this portion is therefore increased dramatically because there is very little space left for the conductive solution. As the overall resistance of the cell 1 is the integral of the resistance along the whole depth of the well 10 and cavity 20 along the axis X, the overall resistance is also increased dramatically. Conversely, if the bead 22 is close to the top surface 4, it occupies a minor proportion of the portion of the well 10 extending around the bead 22. The resistance of this portion is thus less increased than previously, and the overall resistance of the well 10 is smaller than previously.

The resistance of the well 10 thus depends on the distance between the bead 22 and the bottom surface 2, which depends on the length of the biomolecule 24 attached to the bead 22. By measuring the impedance between the top electrode 14 and the bottom electrode 18, it is possible to derive the length of the biomolecule 24.

The top electrode 14 and the bottom electrode 18 are in contact with the electrically conductive solution. One of the electrodes 14, 18 is connected to a voltage source (not shown), preferably an AC voltage source, and is set to reference peak voltage $V_0$ at least intermittently. The reference peak voltage $V_0$ is preferably below 1.2 V, and more preferably below 0.8 V to avoid electrolysis phenomenon within the well 10. For example, the reference peak voltage $V_0$ is comprised between 10 and 500 mV, preferably comprised between 50 and 300 mV. This electrode is used as a reference electrode (preferably the top electrode 14) and may be common to several cells 1, whereas the other electrode used as a measurement electrode (preferably the bottom electrode 18) is specific to each cell 1 or well 10.

An electronic circuit measures the impedance between the two electrodes 14, 18 by measuring the current flowing between them. The electronic circuit may comprise a known resistance connected in series with the measurement electrode. A potential difference at the poles of the resistance may be measured to infer the current flowing through the resistance. Amplification circuitry may be provided to amplify the measurement signal. For sake of simplicity, the electronic circuit and the various electric connections are not represented.

The analysis of the biomolecule 24 is carried out on the basis of the measurement signal. For example, the analysis may comprise the determination of a nucleic acid sequence, i.e. the deciphering of the actual succession of bases in a nucleic acid, but also the determination of other pieces of information on the nucleic acid sequence, such as the detection of a particular sequence in a nucleic acid molecule, the detection of a difference between the sequences of two different nucleic acid molecules, or the binding of a protein to a specific sequence, see e.g. WO 2011/147931 ; WO 2011/147929 ; WO 2013/093005 ; WO 2014/114687. Details can be found in patent EP3090803.

As a non-limiting example, the process can for instance be carried out according to the sequence disclosed in document EP2390351, to which one can refer for more details about the implementation of the sequence. In this example, the biomolecule 24 is a hairpin nucleic acid molecule.

First, the bead is actuated to separate the two strands of the hairpin biomolecule 24, by applying a tension about 15 pN or more on the molecule, for instance equal to 18 pN. The distance between the bead 22 and the bottom surface 2 is derived from the impedance measurement, which corresponds to the total length of the opened hairpin biomolecule 24. A piece of single-stranded nucleic acid molecule is then hybridized with one of the strands of the biomolecule 24.

The bead 22 is then actuated to release the tension applied to the biomolecule 24. The nucleic acid biomolecule 24 then rezips to reform a hairpin. However, the presence of a single-stranded nucleic acid molecule hybridized to one of the nucleic acid strands leads to a pause in re-hybridization (or rezipping) of the hairpin. The detection of such a pause indicates that the single-stranded nucleic acid biomolecule 24 comprises a sequence which is complementary to a part of the hairpin biomolecule 24. Moreover the continuing measurement of the length of the biomolecule 24 during the re-hybridization of the hairpin, including the measurement of the length of the biomolecule 24 during the pause when the hairpin biomolecule 24 is partly re-hybridized, allows determining the position of the said sequence in the biomolecule 24. Indeed, the comparison between the length of the biomolecule 24 at the moment of the pause and the total length of the biomolecule 24 allows inferring the exact position of the hybridized nucleic acid biomolecule 24, from which the sequence of the biomolecule 24 at said position can be deduced.

A measurement signal reflecting accurately the changes of the impedance of the well 10 is therefore of paramount importance for the analysis of the biomolecule 24. Due to the cavity 20, the conductive solution is in contact with the bottom electrode 18 over a large area, larger than the largest cross-section of the well 10. The increased electrode area in contact with the conductive solution leads to a significant increase in the measurement signal's strength, improving the signal to noise ratio. It is then possible to reduce the size of the cell 1 in comparison with a cell 1 without a cavity 20, without having too small a measurement signal. More cells 1 can thus be accommodated in the same volume, greatly improving the throughput of the apparatus without increasing the cost in the same proportion. It is also possible to accelerate the measure since the measurement signal is more stable and accurate values are more easily obtained, thereby increasing the throughput of the apparatus without increasing the cost.

The presence of the cavity 20 makes the manufacturing of the apparatus easier. The shape of the well 10 must be carefully designed, and the manufacturing process must respect the determined shape of the well 10. The shape of the well 10 dictates the relationship between the measurement signal and the position of the bead 22. Any inaccuracy in the dimensions of the well 10 may lead to measurement errors. With known manufacturing techniques, it may be difficult to ensure accurate dimensions of a bottom portion of a well in contact with a bottom electrode. Having the cavity 20 between the bottom electrode 18 and the well 10 allows relaxing the strict design parameters of the well 10 near the bottom electrode 18 since the dimensions of the cavity 20 do not have to be strictly controlled, or at least only global parameters such as cavity height H and diameter are to be considered. Constraining parameters such as the slope of the cavity wall no longer need to be considered. The main function of the cavity 20 is indeed to provide a large contact area between the bottom electrode 18 and the conductive solution, and this function involves few design constraints.

Adding the cavity 20 between the well 10 and the bottom electrode 18 can be made in any way known by the person skilled in the art. Two ways of manufacturing a cell 1 with a cavity 20 between the well 10 and the bottom electrode 18 will now be described. The two examples share many aspects, which can be summarized in the following steps:

a) forming a bottom electrode 18 on a dielectric substrate 8 to form the bottom surface 2,
b) depositing a first layer of a first material onto the bottom electrode 18,
c) depositing a second layer of a second material covering the first layer, the second material different from the first material,
d) forming the well 10 in the second layer of second material, thereby exposing an area of first material,
e) forming the cavity 20 by etching first material through the well 10,
f) forming the top electrode 14,
g) filling the well 10 and cavity with the electrically conductive solution, anchoring the biomolecule 24 at a first end to the bottom surface 2 and at a second end to the bead 22.

The first material is an electrically insulating material, for example a semiconductor such as silicon, or insulator such as silicon dioxide, silicon nitride, glass, a non-conducting polymer such as polyimide, an organic dielectric, or a resin. The thickness (or height) of the first layer 30 is chosen to correspond to the desired height (or depth) of the cavity 20.

The second material is also an electrically insulating material, and can be a semiconductor such as silicon, or insulator such as silicon dioxide, silicon nitride, glass, a non-conducting polymer such as polyimide, an organic dielectric, or a resin. The first material and the second material are chosen so as to be able to selectively etch the first material without altering the second material, one example being a first material of silicon dioxide and a second material of polyimide, where the first material can be etched selectively from the second material using hydrofluoric acid solution.

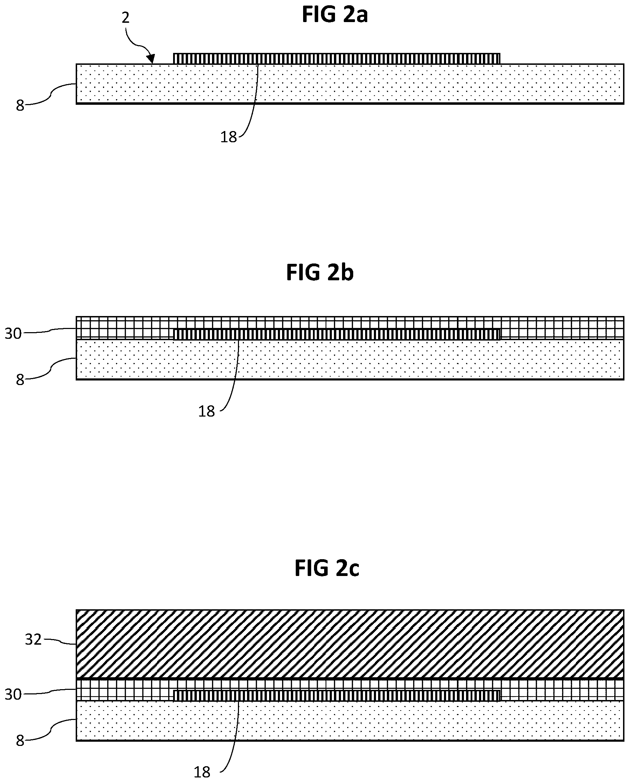

With reference to **Figure 2a**, a first process for manufacturing an apparatus first involves the step a) of forming a bottom electrode 18 on a dielectric substrate 8. The dielectric substrate 8 can be for example glass, or a silicon wafer with an insulating coating such as silicon dioxide or silicon nitride or combination thereof. As mentioned above, the bottom electrode 18 may be made of gold or platinum deposited on the substrate through conventional deposition techniques such as electron beam evaporation or sputtering. The bottom surface 2 is thereby formed by the upper surface of the dielectric substrate 8 and the bottom electrode 18 thereon.

In step b) illustrated in **Figure 2b**, a first layer 30 of a first material is deposited onto the bottom electrode 18 and the surface of the substrate 8 other than the bottom electrode 18. In step c) illustrated in **Figure 2c**, a second layer 32 of a second material is deposited to cover the first layer 30.

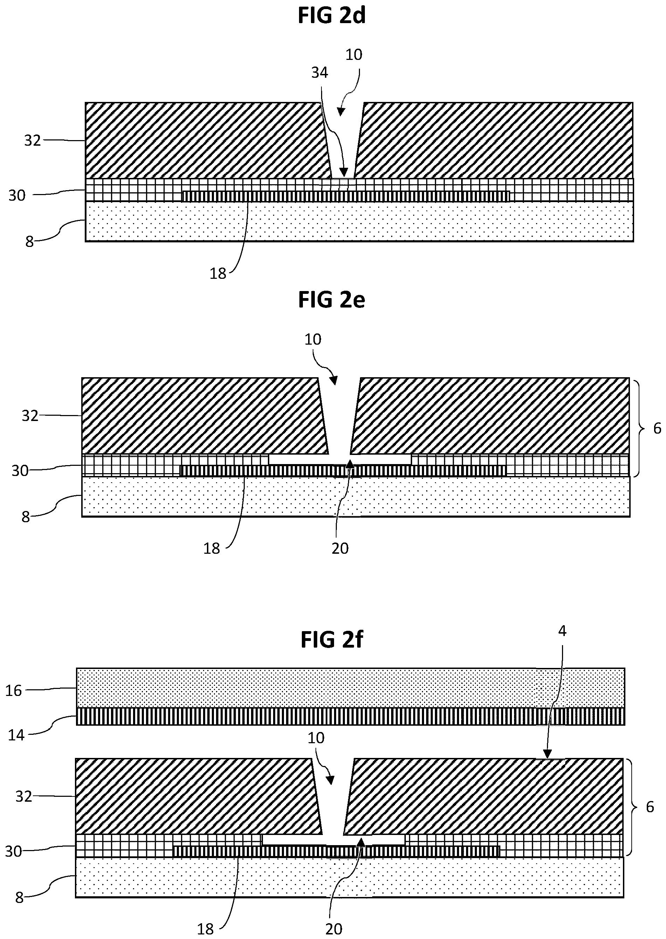

In step d) illustrated in **Figure 2d**, the well 10 is formed in the second layer 32 of second material, thereby exposing an area 34 of first material at the bottom of the well 10. The well 10 is formed for example by transfer of reflow resist pattern, or dry etching with control of sidewall angle, or nano imprint lithography.

In step e) illustrated in **Figure 2e**, the cavity 20 is formed by etching first material through the well 10. The etching starts by removing the first material from the area 34 of first material exposed by the well 10. The first material is removed on all the thickness of the first layer 30. A pre-cavity is formed in the first layer 30, with a cross-section that increases as the etching goes on. When the cavity 20 of desired dimensions is obtained, the etching is stopped. The dimensions of the cavity 20 can therefore be controlled by the duration of the etching. The remaining of the first layer 30 is kept. The cavity 20 is defined in the first layer 30 of first material. The first layer 30 and the second layer 32 form together the main layer 6 of electrically insulating material.

Since the first material is etched through the well 10, the cavity 20 thus formed is automatically aligned with the well 10. This approach allows a shorter process, with fewer steps, in particular few alignment steps. The duration of the etching must however be precisely controlled. Variations between cavity sizes of different cells may also appear.

In step f) illustrated by **Figure 2f**, the top electrode 14 is arranged, for example by depositing metal such as gold on a support plate 16 and bringing the support plate 16 above the well 10, at a distance of the top surface 4 to leave room for the channel 12. In a further step g) the well 10 and cavity 20 are filled with the electrically conductive solution, the biomolecule 24 is anchored at a first end to the bottom surface 2 and at a second end to the bead 22, to obtain a cell 1 as illustrated in **Figure 1.**

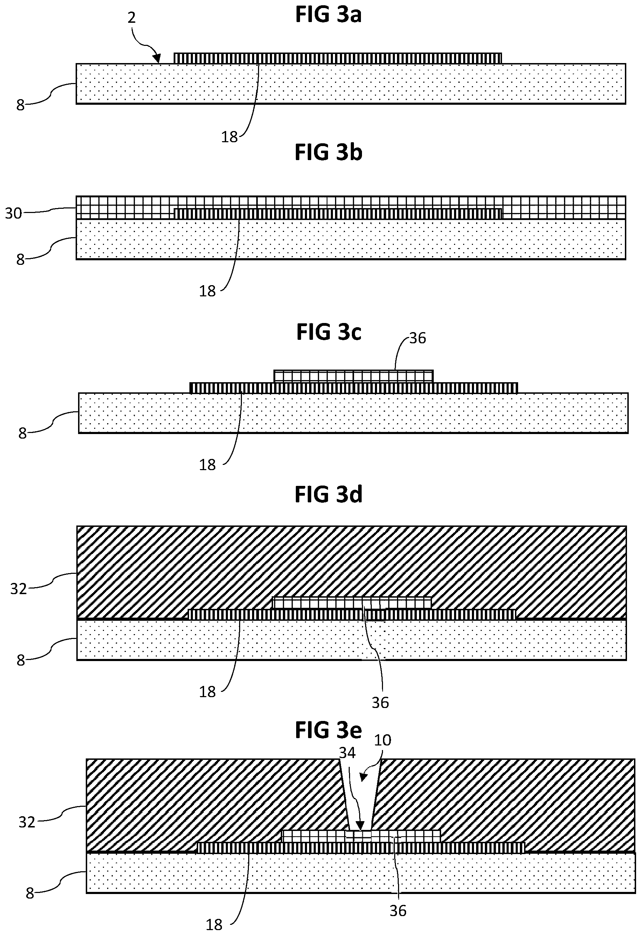

With reference to **Figure 3a,** a second process for manufacturing an apparatus first involves the step a) of forming a bottom electrode 18 on a dielectric substrate 8. The dielectric substrate 8 can be for example glass, a silicon wafer with an insulating coating such as silicon dioxide or silicon nitride or combination thereof. As mentioned above, the bottom electrode 18 may be made of gold or platinum deposited on the substrate through conventional deposition techniques such as electron beam evaporation or sputtering. The bottom surface 2 is formed by the upper surface of the dielectric substrate 8 and the bottom electrode 18 thereon.

In step b) illustrated in **Figure 3b,** a first layer 30 of a first material is deposited on the bottom electrode 18. The first layer 30 is also deposited on parts of the bottom surface 2 of the substrate 8 other than the bottom electrode 18. The first layer 30 thus covers the entire bottom surface 2.

In a step b1) following the step b) of depositing a first layer of a first material onto the bottom electrode 18 and illustrated by **Figure 3c,** the first material of the first layer 30 is removed except for a volume 36 of first material above the bottom electrode 18. The volume 36 of first material which is left correspond to the desired volume of the cavity 20. The first layer 30 of first material now consists in the remaining volume 36 of first material. The removal of the first material of the first layer 30 is for example performed by wet etching or dry etching using standard techniques with appropriate selectivity well known to those skilled in the art.

In step c) illustrated by **Figure 3d,** a second layer 32 of a second material is deposited to cover the first layer 30, which is now restricted to the remaining volume 36 of first material in this embodiment. The second layer 32 also covers the bottom surface 2 where the first material was previously removed.

In step d) illustrated by **Figure 3e,** the well 10 is formed in the second layer 32 of second material, thereby exposing an area 34 of first material of the volume 36 of first material above the bottom electrode 18. The well 10 is formed for example by transfer of reflow resist pattern, or dry etching with control of sidewall angle, or nano imprint lithography.

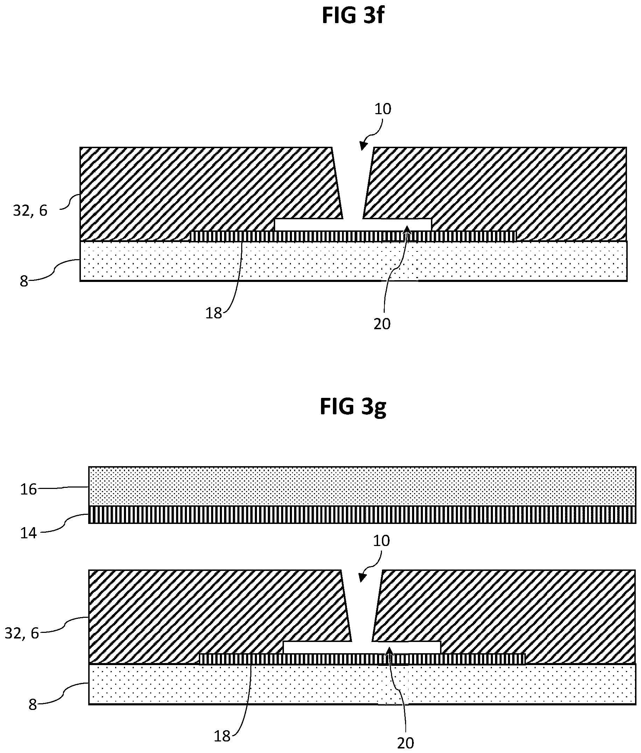

In step e) illustrated in **Figure 3f,** the cavity 20 is formed by etching first material through the well 10. The etching starts by removing the first material from the area 34 of first material exposed by the well 10, and is carried out until the volume 36 of first material above the bottom electrode 18 is entirely removed, thus leaving the cavity 20. Dimensions of the cavity are defined by the volume 36 of first material left by the removal of the first material of the first layer 30 in step b1). Consequently, the size and shape of the cavity 20 is defined before the etching, which lead to an etching process extremely tolerant to process variations, especially regarding duration variations. In this embodiment, the entirety of the first material is removed either during step b1) or step e). The main layer 6 of electrically insulating material is therefore constituted only of the second layer 32 of second material.

In this approach, the cavity 20 is defined in the second layer 32 of second material, since the first layer 30 no longer exists. The dimensions of the cavity 20 are more precisely defined, which improves cavity size uniformity between different cells. Also, the etching is much easily controlled since the cavity size is not defined by the etching duration. This approach however requires an additional alignment step between the well 10 and the volume 36 of first material.

In step f) illustrated by **Figure 3g,** the top electrode 14 is arranged, for example by depositing metal such as gold on a support plate 16 and bringing the support plate 16 above the well 10, at a distance of the top surface 4 to leave room for the channel 12. In a further step g) the well 10 and cavity 20 are filled with the electrically conductive solution, the biomolecule 24 is anchored at a first end to the bottom surface 2 and at a second end to the bead 22, to obtain a cell 1 as illustrated in **Figure 1.**

While the present invention has been described with respect to certain preferred embodiments, it is obvious that it is in no way limited thereto and it comprises all the technical equivalents of the means defined in the appended claims. In particular, it will be apparent to those skilled in the art that various changes and modifications may be made without departing from the scope of the invention as defined in the appended claims.

## Claims

1. An apparatus for biomolecule analysis, comprising a plurality of cells (1), each cell (1) comprising:

   - a bottom surface (2) and a top surface (4) opposite the bottom surface (2), an axis (X) extending between the bottom surface (2) and the top surface (4);
   - a well (10) extending along the axis (X) from the top surface (4) towards the bottom surface (2), wherein the well (10) has a cross-sectional area perpendicular to the axis (X), which monotonically varies along the axis of the well (10) between a minimum and a maximum;
   - a molecule (24) anchored at a first end to the bottom surface (2);
   - a bead (22) to which a second end of the biomolecule is anchored, the bead (22) disposed within the well (10) at a position;
   - a electrically conductive solution filling the well (10);
   - a top electrode (14) above the well, and a bottom electrode (18) at the bottom surface (2), both the top electrode (14) and the bottom electrode (18) in contact with the electrically conductive solution,

      wherein the apparatus comprises a sensor configured to measure an impedance between the top electrode (14) and the bottom electrode (18), the impedance depending on the depth of the bead (22) within the well (10),

**characterized in that** a cavity (20) wider than the well (10) is arranged between the bottom electrode (18) and the well (10), said cavity (20) in fluid communication with the well (10) and exposing the bottom electrode (18) at the bottom surface to the electrically conductive solution filling the cavity (20).

2. The apparatus of claim 1, wherein the well (10) is at least twice as deep as the cavity (20) along the axis (X).

3. The apparatus of claim 1, wherein the cross-sectional area of the well (10) decreases in a direction from the top surface (4) to the bottom surface (2) until a minimum is reached at a transition between the well (10) and the cavity (20).

4. The apparatus of any one of claims 1 to 3, wherein a cross-sectional area of the cavity (20) is at least twice a maximum cross-sectional area of the well (10).

5. The apparatus of any one of claims 1 to 4, wherein the well (10) is rotationally symmetrical about the axis (X) and the cavity (20) has a cylindrical shape.

6. The apparatus of any one of claims 1 to 5, wherein the cavity (20) has a height along the axis (X) comprised between 0.05 $\mu$m and 1 $\mu$m.

7. The apparatus of any one of claims 1 to 6, wherein the apparatus comprises an actuator configured to cause the bead (22) to move relative to the bottom surface (2) along the axis (X) of the well (10).

8. A process for manufacturing an apparatus as claimed in any one of the preceding claims, comprising:

a) forming a bottom electrode (18) on a dielectric substrate (8) to form the bottom surface (2),
b) depositing a first layer (30) of a first material onto the bottom electrode (18),
c) depositing a second layer (32) of a second material covering the first material of the first layer (30), the second material different from the first material,
d) forming the well (10) in the second layer (32) of second material, thereby exposing an area (34) of first material,
e) forming the cavity (20) by etching first material through the well (10),
f) forming the top electrode (14),
g) filling the well (10) and cavity (20) with the electrically conductive solution, anchoring the biomolecule (24) at a first end to the bottom surface (2) and at a second end to the bead (22).

9. The process of claim 8, wherein step b) of depositing a first layer (30) of a first material on the bottom electrode (18) is followed by a step b1) in which the first material of the first layer is removed except for a volume (36) of first material above the bottom electrode (18), and wherein at step e) said volume (36) of first material is etched to form the cavity (20).

10. The process of claim 8, wherein dimensions of the cavity are defined by the volume (36) of first material left by the removal of the first material of the first layer in step b1).

11. The process of any one of claims 8 to 10, wherein the cavity (20) is defined in the second layer (32) of second material.

12. The process of claim 8, wherein dimensions of the cavity (30) are defined by a duration of the etching of the first material in step e).

13. The process of claim 12, wherein the cavity (20) is defined in the first layer (30).

**Patentansprüche**

1. Vorrichtung zur Biomolekülanalyse, umfassend eine Vielzahl von Zellen (1), wobei jede Zelle (1) Folgendes umfasst:

- eine untere Fläche (2) und eine obere Fläche (4), die der unteren Fläche (2) gegenüberliegt, wobei sich eine Achse (X) zwischen der unteren Fläche (2) und der oberen Fläche (4) erstreckt;
- ein Vertiefung (10), die sich entlang der Achse (X) von der oberen Fläche (4) in Richtung der unteren Fläche (2) erstreckt, wobei die Vertiefung (10) eine Querschnittsfläche senkrecht zu der Achse (X) aufweist, welche entlang der Achse der Vertiefung (10) monoton zwischen einem Minimum und einem Maximum variiert;
- ein Molekül (24), das an einem ersten Ende der unteren Fläche (2) verankert ist;
- ein Kügelchen (22), an dem ein zweites Ende des Biomoleküls verankert ist, wobei das Kügelchen (22) innerhalb der Vertiefung (10) an einer Position angeordnet ist;
- eine elektrisch leitende Lösung, die die Vertiefung (10) füllt;
- eine obere Elektrode (14) über der Vertiefung und eine untere Elektrode (18) an der unteren Fläche (2), wobei sowohl die obere Elektrode (14) als auch die untere Elektrode (18) in Kontakt mit der elektrisch leitenden Lösung stehen,

wobei die Vorrichtung einen Sensor umfasst, der dafür konfiguriert ist, eine Impedanz zwischen der oberen Elektrode (14) und der unteren Elektrode (18) zu messen, wobei die Impedanz von der Tiefe des Kügelchens (22) innerhalb der Vertiefung (10) abhängt,

**dadurch gekennzeichnet, dass** ein Hohlraum (20), der breiter als die Vertiefung (10) ist, zwischen der unteren Elektrode (18) und der Vertiefung (10) angeordnet ist, wobei der Hohlraum (20) in Fluidverbindung mit der Vertiefung (10) steht und die untere Elektrode (18) an der unteren Fläche der elektrisch leitenden Lösung aussetzt, die den Hohlraum (20) füllt.

2. Vorrichtung nach Anspruch 1, wobei die Vertiefung (10) entlang der Achse (X) mindestens doppelt so tief wie der Hohlraum (20) ist.

3. Vorrichtung nach Anspruch 1, wobei die Querschnittsfläche der Vertiefung (10) in einer Richtung von der oberen Fläche (4) zu der unteren Fläche (2) abnimmt, bis an einem Übergang zwischen der Vertiefung (10) und dem Hohlraum (20) ein Minimum erreicht ist.

4. Vorrichtung nach einem der Ansprüche 1 bis 3, wobei eine Querschnittsfläche des Hohlraums (20) mindestens doppelt so groß ist wie eine maximale Querschnittsfläche der Vertiefung (10).

5. Vorrichtung nach einem der Ansprüche 1 bis 4, wobei die Vertiefung (10) rotationssymmetrisch um die Achse (X) ist und der Hohlraum (20) eine zylindrische Form aufweist.

6. Vorrichtung nach einem der Ansprüche 1 bis 5, wobei der Hohlraum (20) eine Höhe entlang der Achse (X) zwischen 0,05 $\mu$m und 1 $\mu$m aufweist.

7. Vorrichtung nach einem der Ansprüche 1 bis 6, wobei die Vorrichtung einen Aktor umfasst, der dafür konfiguriert ist zu bewirken, dass sich das Kügelchen (22) entlang der Achse (X) der Vertiefung (10) relativ zu der unteren Fläche (2) bewegt.

8. Verfahren zur Herstellung einer Vorrichtung nach einem der vorstehenden Ansprüche, umfassend:

   a) Bilden einer unteren Elektrode (18) auf einem dielektrischen Substrat (8), um die untere Fläche (2) zu bilden,
   b) Abscheiden einer ersten Schicht (30) aus einem ersten Material auf die untere Elektrode (18),
   c) Abscheiden einer zweiten Schicht (32) aus einem zweiten Material, das das erste Material der ersten Schicht (30) bedeckt, wobei sich das zweite Material von dem ersten Material unterscheidet,
   d) Bilden der Vertiefung (10) in der zweiten Schicht (32) aus dem zweiten Material, wodurch ein Bereich (34) aus dem ersten Material freigelegt wird,
   e) Bilden des Hohlraums (20) durch Ätzen des ersten Materials durch die Vertiefung (10),
   f) Bilden der oberen Elektrode (14),
   g) Füllen der Vertiefung (10) und des Hohlraums (20) mit der elektrisch leitenden Lösung, Verankern des Biomoleküls (24) an einem ersten Ende an der unteren Fläche (2) und an einem zweiten Ende an dem Kügelchen (22) .

9. Verfahren nach Anspruch 8, wobei auf den Schritt b) des Abscheidens einer ersten Schicht (30) aus einem ersten Material auf der unteren Elektrode (18) ein Schritt b1) folgt, in dem das erste Material der ersten Schicht mit Ausnahme eines Volumens (36) des ersten Materials über der unteren Elektrode (18) entfernt wird, und wobei bei dem Schritt e) das Volumen (36) aus dem ersten Material geätzt wird, um den Hohlraum (20) zu bilden.

10. Verfahren nach Anspruch 8, wobei die Abmessungen des Hohlraums durch das Volumen (36) des ersten Materials definiert sind, das nach dem Entfernen des ersten Materials der ersten Schicht in Schritt b1) übrigbleibt.

11. Verfahren nach einem der Ansprüche 8 bis 10, wobei der Hohlraum (20) in der zweiten Schicht (32) aus dem zweiten Material definiert ist.

12. Verfahren nach Anspruch 8, wobei die Abmessungen des Hohlraums (30) durch die Dauer des Ätzens des ersten Materials in Schritt e) definiert werden.

13. Verfahren nach Anspruch 12, wobei der Hohlraum (20) in der ersten Schicht (30) definiert ist.

**Revendications**

1. Appareil pour l'analyse de biomolécules, comprenant une pluralité de cellules (1), chaque cellule (1) comprenant :

   - une surface inférieure (2) et une surface supérieure (4) opposée à la surface inférieure (2), un axe (X) s'étendant entre la surface inférieure (2) et la surface supérieure (4) ;
   - un puits (10) s'étendant le long de l'axe (X) de la surface supérieure (4) vers la surface inférieure (2), dans lequel le puits (10) a une surface de section transversale perpendiculaire à l'axe (X), qui varie de façon monotone le long de l'axe du puits (10) entre un minimum et un maximum ;
- une molécule (24) ancrée à une première extrémité à la surface du fond (2) ;
- une bille (22) à laquelle une deuxième extrémité de la biomolécule est ancrée, la bille (22) étant disposée à l'intérieur du puits (10) à une position ;
- une solution électriquement conductrice remplissant le puits (10) ;
- une électrode supérieure (14) au-dessus du puits, et une électrode inférieure (18) à la surface du fond (2), l'électrode supérieure (14) et l'électrode inférieure (18) étant en contact avec la solution électriquement conductrice,

dans lequel l'appareil comprend un capteur configuré pour mesurer une impédance entre l'électrode supérieure (14) et l'électrode inférieure (18), l'impédance dépendant de la profondeur de la bille (22) dans le puits (10),
**caractérisé en ce qu'une** cavité (20) plus large que le puits (10) est disposée entre l'électrode inférieure (18) et le puits (10), ladite cavité (20) étant en communication fluidique avec le puits (10) et en exposant l'électrode inférieure (18) à la surface inférieure de la solution électriquement conductrice remplissant la cavité (20).

2.  Appareil selon la revendication 1, dans lequel le puits (10) est au moins deux fois plus profond que la cavité (20) le long de l'axe (X).

3.  Appareil selon la revendication 1, dans lequel la section transversale du puits (10) diminue dans une direction allant de la surface supérieure (4) à la surface inférieure (2) jusqu'à ce qu'un minimum soit atteint à un point de transition entre le puits (30) et la cavité (20).

4.  Appareil selon l'une des revendications 1 à 3, dans lequel une section transversale de la cavité (20) est au moins deux fois supérieure à la section maximale du puits (10).

5.  Appareil selon l'une des revendications 1 à 4, dans lequel le puits (10) présente une symétrie de rotation par rapport à l'axe (X) et la cavité (20) a une forme cylindrique.

6.  Appareil selon l'une des revendications 1 à 5, dans lequel la cavité (20) a une hauteur le long de l'axe (X) compris entre 0,05 $\mu$m et 1 $\mu$m.

7.  Appareil selon l'une des revendications 1 à 6, dans lequel l'appareil comprend un actionneur configuré pour provoquer le déplacement du bourrelet (22) par rapport à la surface inférieure (2) le long de l'axe (X) du puits (10).

8.  Procédé de fabrication d'un appareil selon l'une quelconque des revendications précédentes, comprenant :

    a) former une électrode inférieure (18) sur un substrat diélectrique (8) pour former la surface inférieure (2),
    b) déposer une première couche (30) d'un premier matériau sur l'électrode inférieure (18),
    c) déposer une deuxième couche (32) d'un deuxième matériau recouvrant le premier matériau de la première couche (30), le deuxième matériau étant différent du premier,
    d) former le puits (10) dans la seconde couche (32) du second matériau, exposant ainsi une zone (34) du premier matériau,
    e) former la cavité (20) en gravant le premier matériau à travers le puits (10),
    f) former l'électrode supérieure (14),
    g) remplir le puits (10) et la cavité (20) avec la solution électriquement conductrice, ancrer la biomolécule (24) à une première extrémité à la surface inférieure (2) et à une seconde extrémité à la perle (22).

9.  Procédé selon la revendication 8, dans lequel l'étape b) de dépôt d'une première couche (30) d'un premier matériau sur l'électrode inférieure (18) est suivie d'une étape b1) dans laquelle le premier matériau de la première couche est enlevé à l'exception d'un volume (36) de premier matériau au-dessus de l'électrode inférieure (18), et dans lequel à l'étape e) ledit volume (36) de premier matériau est gravé pour former la cavité (20).

10. Procédé selon la revendication 8, dans lequel les dimensions de la cavité sont définies par le volume (36) de premier matériau laissé par l'enlèvement du premier matériau de la première couche à l'étape b1).

11. Procédé selon l'une des revendications 8 à 10, dans lequel la cavité (20) est définie dans la deuxième couche (32) du deuxième matériau.

12. Procédé selon la revendication 8, dans lequel les dimensions de la cavité (30) sont définies par une durée de la gravure du premier matériau à l'étape e).

13. Procédé selon la revendication 12, dans lequel la cavité (20) est définie dans la première couche (30).

# FIG 1

**FIG 2a**

**FIG 2b**

**FIG 2c**

# FIG 2d

# FIG 2e

# FIG 2f

## FIG 3a

## FIG 3b

## FIG 3c

## FIG 3d

## FIG 3e

# FIG 3f

32, 6

8

18

20

10

# FIG 3g

16

14

32, 6

8

18

20

10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3090803 A
- WO 2011147931 A
- WO 2011147929 A
- WO 2013093005 A
- WO 2014114687 A
- EP 2390351 A

**Non-patent literature cited in the description**

- **HUNTER MM** ; **MARGOLIES MN** ; **JU A** ; **HABER E**. High-affinity monoclonal antibodies to the cardiac glycoside, digoxin. *Journal of Immunology*, September 1982, vol. 129 (3), 1165-1172
- **G. SITTERS** ; **D. KAMSMA** ; **G. THALHAMMER** ; **M. RITSCH-MARTE** ; **E. J. G. PETERMAN** ; **G. L. J. WUITE**. Acoustic Force spectroscopy. *Nature Methods*, January 2015, vol. 12 (1)
- **X. DING** ; **Z. S. LIN** ; **B. KIRALY** ; **H. YUE** ; **S. LI** ; **I. CHIANG** ; **J. SHI** ; **S. J. BENKOVIC** ; **T. J. HUANG**. On-Chip Manipulation of single microparticles, cells, and organisms using surface acoustic waves. *PNAS*, 10 July 2012, vol. 109 (28), 11105-11109